# EUROPEAN PATENT SPECIFICATION

(11) **EP 3 041 586 B1**
(45) Date of publication and mention of the grant of the patent: **30.11.2016**
(21) Application number: 14742201.8
(22) Date of filing: 21.07.2014
(51) Int. Cl.: A61Q 19/08, A61K 8/97, A23L 2/02

(54) **SKIN CARE COMPOSITION**
HAUTPFLEGEZUSAMMENSETZUNG
COMPOSITION DE SOIN DE LA PEAU

(30) Priority: 09.08.2013 EP 13179839
(43) Date of publication of application: 13.07.2016
(73) Proprietor: Unilever N.V., 3013 AL Rotterdam (NL); Unilever PLC, London, Greater London EC4Y 0DY (GB)
(72) Inventor: DIAS, Paul Mark, Bangalore 560 066 (IN); GADGIL, Vijay Ramchandra, Bangalore 560 066 (IN); LAHORKAR, Praful Gulab Rao, Bangalore 560 066 (IN); SHUKLA, Ravi Kant, Bangalore 560 066 (IN)
(74) Representative: Warner, Guy Jonathan
(86) International application number: PCT/EP2014/065610
(87) International publication number: WO 2015/018631

(56) References cited:
- DATABASE GNPD [Online] MINTEL; November 2012 (2012-11), "Peedantak Taila", XP002720804, Database accession no. 1918921
- DATABASE GNPD [Online] MINTEL; October 2010 (2010-10), "Ayurvedic Skincare", XP002720806, Database accession no. 1420678
- DATABASE GNPD [Online] MINTEL; November 2010 (2010-11), "Saffron Beauty Blend Ayurvedic Serum", XP002720805, Database accession no. 1442368
- KROLIKIEWICZ-RENIMEL ISABELLE ET AL: "Protective effect of aButea monosperma(Lam.) Taub. flowers extract against skin inflammation: Antioxidant, anti-inflammatory and matrix metalloproteinases inhibitory activities", JOURNAL OF ETHNOPHARMACOLOGY, vol. 148, no. 2, 13 May 2013 (2013-05-13), pages 537-543, XP028571868, ISSN: 0378-8741, DOI: 10.1016/J.JEP.2013.05.001

## Description

### Field of the invention

The invention relates to a composition that provides enhanced blood macrocirculation or blood microcirculation when applied on skin or is ingested. This composition has applications in areas such as reduction of under-eye dark circles, scalp health, reduction in under-arm darkening when applied topically and provides reduction in cardiovascular diseases when ingested orally. In both cases there is anti-inflammatory benefits and other skin health benefits including anti-aging and skin lightening.

### Background of the invention

Life-style modification and age increases the probability to acquire major health problems like cardiovascular risk factors. Impairment in blood vessel flexibility is a major risk factor for the onset of such diseases, reflected as hypertension, angina and atherosclerosis. In humans, the investigation of endothelial function has centered on either the macrocirculation or the microvasculature. Microcirculation is the delivery of fresh blood to the smallest blood vessels, present in the vasculature embedded within organ tissues. This contrasts with macrocirculation, which transport blood to and from the organs. The main functions of the microcirculation include 1. regulation of blood flow and tissue perfusion 2. regulation of blood pressure, 3. regulation of tissue fluid (swelling or edema), 4. delivery of oxygen and other nutrients and removal of carbon dioxide and other metabolic waste products, 5. regulation of body temperature, and 6. reduction of sedentary aging (wrinkles, under eye dark circles). In addition to maintaining these functions for overall health benefits, microcirculation enhances skin health.

Skin microcirculation is a complex and dynamic system which is important for thermoregulation, skin metabolism and trans-cutaneous penetration. The blood supply to the skin is provided by a network of arterioles, capillaries and venules organized into a superficial and a deep plexus. Skin is exposed to environmental stressors (such as UV, chemical pollutants and particulate matter) or physiological (non-environmental) stressors (psychological stress, sedentary aging and inflammation). Such compromised blood flow leads to physiological effects such as under-eye dark circles, wrinkles, retarded wound healing and edema.

The present inventors have been looking for solution to problems like reduction of under-eye dark circles and overall skin health through compositions that may be applied topically or ingested orally. The inventors wish to achieve this using actives (or combination of actives) that may be present in nature in abundance. They have found that a combination of an extract of *Rubia Cordifolia* comprising higher than 0.025% hydroxyanthraquinones and hydroxynaphthoquinones and an extract of *Butea monosperma* comprising higher than 0.02 % Butein when applied topically or ingested provides for the enhanced microcirculation thereby giving the above benefits.

*Rubia cordifolia* and *Butea monosperma* have both been used individually for topical application but not together in a single composition.

US2008206373 discloses a personal care composition comprising at least one extract selected from the group consisting of extracts of *Terminalia bellerica, Butea monosperma, Mallotus philippinensis, Anogeissus latifolia, Innula racemosa, Ficus benghalensis, Nerium indicum, Psoralea corylifolia, Acacia catechu, Abies pindrow, Cedrus deodara, Emblica officinalis, Moringa oleifera, Glycyrrhiza glabra,* and mixtures thereof, and a dermatologically acceptable carrier.

KR20090119340 discloses a cosmetic composition for skin whitening containing *Rubia cordifolia* extract to ensure the effect of suppressing melanogenesis and tyrosinase activation which has applications in skin lightening.

Indian patent application 1099/MUM/2008 discloses *Rubia cordifolia* plant extracts containing anthraquinone derivatives which are assessed to have antimicrobial activity and also provides anti-inflammatory and anti-irritant properties.

It is heretofore not known that combination of extract of *Rubia cordifolia* and *Butea monosperma* provides for enhanced microcirculation which gives benefits in reduction of under-eye dark circles, scalp health, reduction in under-arm darkening when applied topically and exhibits reduction in cardiovascular diseases when ingested orally.

### Summary of the invention

The present invention provides for a topical, oral or parenteral composition for enhanced micro/ macro blood circulation comprising
(i) 0.1 to 10 % of an extract *of Rubia cordifolia* comprising higher than 0.025 % hydroxyanthraquinones and hydroxynaphthoquinones;
(ii) 0.1 to 10 % an extract of *Butea monosperma* comprising higher than 0.02 % butein.

It is preferred that the composition comprises 0.1 to 5% by weight of an extract of *Rubia cordifolia* and 0.3 to 3% by weight of an extract of *Butea monosperma.*

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Numerical ranges expressed in the format "from x to y" are understood to include x and y. When for a specific feature multiple preferred ranges are described in the format "from x to y", it is understood that all ranges combining the different endpoints are also contemplated. "Topical composition" as used herein, is meant to include a composition for application to the external surface e.g. skin of mammals, especially humans for better skin health benefits. Such a composition may be generally classified as leave-on or rinse off, preferably leave-on and includes any product applied to a human body primarily for enhanced health of skin but may be used also for improving appearance, cleansing, odour control or general aesthetics. The composition of the present invention can be in the form of a liquid, an emulsion, lotion, cream, foam, gel, soap bar, stick, bar, pad or patch. Non-limiting examples of such topical compositions include leave-on skin lotions and creams, antiperspirants, deodorants, lipsticks, foundations, mascara, sunless tanners or sunscreen lotions, and wash-off products like shampoos, conditioners, shower gels, or toilet bars. "Skin" as used herein is meant to include skin on the face and body (e.g., neck, chest, back, arms, underarms, hands, legs, buttocks and scalp) and especially to the under-eye portions of the face. The topical composition of the invention is especially useful for application on skin areas that get wrinkled or are more likely to get wrinkled especially to the sun exposed parts of the body.

By a food product is meant a product for oral ingestion or intravenous injection into mammals particularly humans. Food products introduced into the human body through the oral ingestion route are especially preferred. Food products also known as edible products which are especially preferred for delivery of the synergistic combination of the present invention can be in the form of a liquid such as a soup or a beverage like tea or coffee or in non-liquid forms like a spread, a dressing, a dessert or bread. Delivery through a beverage like a tea based beverage is especially preferred. The edible product may be in the form of a solid or powdered food supplement.

The composition comprises an extract of *Rubia cordifolia* and *Butea monosperma.* An aqueous extract is especially preferred. An aqueous extract, as per this invention, is an extract of a plant source which has been first extracted with water. This extract may however, be further fractionated with other solvents. The composition comprises 0.1 to 10% of an extract of *Rubia cordifolia* comprising higher than 0.025% hydroxyanthraquinones and hydroxynaphthoquinones. Hydroxyanthraquinones have the general structure

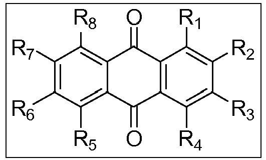

In the above structure, the pendant groups R1 to R8 is preferably selected from the groups given below:
R₁, R₄, R₅ and R₈ are selected from one of H or OH
R₂ is selected from H, OH, CH₃ or COOH
R₃ and R₆ are selected from one of H, OH or CH₂OH
R7 is selected from H, CH3 or OCH3 and
R8 is H.

Furthermore preferably the hydroxyanthraquinones are preferably one or more of the following:

| S.No. | | R₁ | R₂ | R₃ | R₄ | R₅ | R₆ | R₇ | R₈ |
|---|---|---|---|---|---|---|---|---|---|
| 1 | 1-hydroxy-2-methyl AQ | OH | CH₃ | H | H | H | H | H | H |
| 2 | 4,5-dihydroxy-2-methyl AQ | H | CH₃ | H | OH | OH | H | H | H |
| 3 | 4,5-dihydroxy--7-methoxy -2-methyl AQ | H | CH₃ | H | OH | OH | H | OCH3 | H |
| 4 | 4-hydroxy-2-methyl AQ | H | CH₃ | H | OH | H | H | H | H |
| 5 | 1,5-dihydroxy-2-methyl AQ | OH | CH₃ | H | H | OH | H | H | H |
| 6 | 1,5-dihydroxy-2-fromyl AQ | OH | CHO | H | H | OH | H | H | H |
| 7 | 1,3-dihydroxy-2-carboxyl AQ | OH | COOH | OH | H | H | H | H | H |
| 8 | 1,3,4-trihydroxy2carboxylAQ | OH | COOH | OH | OH | H | H | H | H |
| 9 | 2,3-dihydroxy AO | H | OH | OH | H | H | H | H | H |
| 10 | 1,3-dihydroxy- AQ | OH | H | OH | H | H | H | H | H |
| 11 | 1,2,3-trihydroxy AQ | OH | OH | OH | H | H | H | H | H |
| 12 | 1,hydroxy-2-7-dimethy AQ | OH | CH₃ | H | H | H | H | CH₃ | H |
| 13 | 2-hydroxy-6-methyl AQ | H | OH | H | H | H | CH₃ | H | H |
| 14 | 2,6-dihydroxy- AQ | H | OH | H | H | H | OH | H | H |
| 15 | 1,2,4-trihydroxy AQ | OH | OH | H | OH | H | H | H | H |
| 16 | 2,5-dihydroxy- AQ | H | OH | H | H | OH | H | H | H |
| 17 | 1,2-dihydroxy- AQ | OH | OH | H | H | H | H | H | H |
| 18 | 1,3-dihydroxy-2-methyl AQ | OH | CH₃ | OH | H | H | H | H | H |
| 19 | 1,3, 8-trihydroxy-6-methyl AQ | OH | H | OH | H | H | CH₃ | H | OH |
| 20 | 1,8-dihydoroxy-3-hydroxymethyl AQ | OH | H | CH₂OH | H | H | H | H | OH |

| | | | | | | | | | |
|---|---|---|---|---|---|---|---|---|---|
| AQ in the above table refers to anthraquinone. | | | | | | | | | |

The preferred hydroxynaphthoquinones present in the extract of *Rubia cordifolia* for use in the composition of the invention are

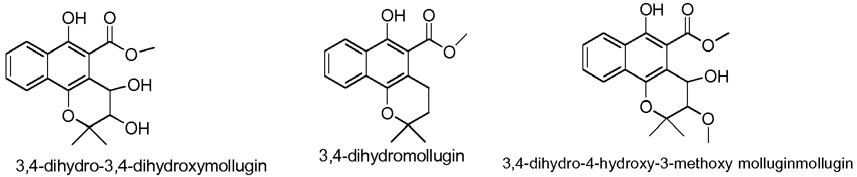

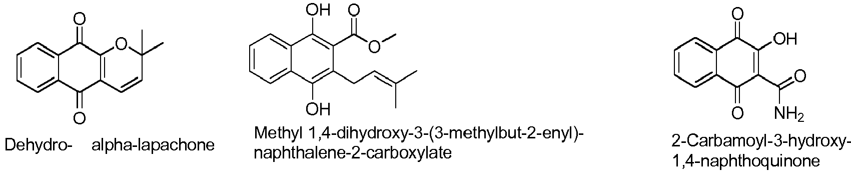

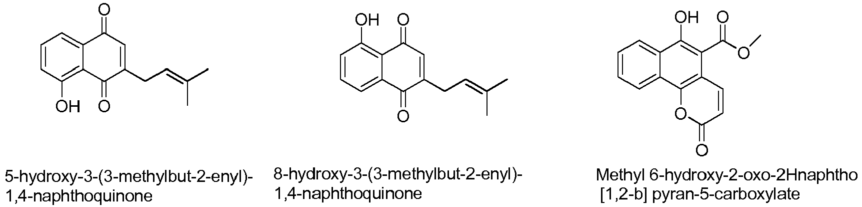

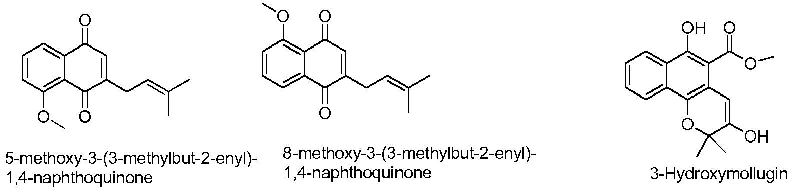

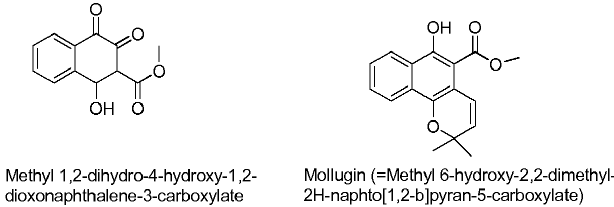

*Rubia cordifolia Linn* (Rubiaceae) is a perennial, herbaceous, slender, branched; climbing plant, with very long cylindrical roots, flexuous, with a thin red bark widely distributed in India, China, temperate Asia, Africa and tropical Australia. *Rubia cordifolia* has very high commercial and medicinal importance. Medicinally, *Rubia cordifolia* is important as the source of actives for various prescriptions in Ayurveda, traditional Chinese medicine system and other modern drugs. In the present invention, the stem of the plant is preferred for use in the composition.

The extract of *Rubia Cordifolia* for use in the present invention is preferably extracted using one of two extraction processes to prepare two types of extracts both of which are basically aqueous extracts: (i) Aqueous Extract or (ii) Phenolic fraction of aqueous extract.

### Preferred process for preparation of aqueous extract of Rubia cordifolia:

The stems of *Rubia cordifolia* were powdered and extracted with distilled water (1:10 by w/v) by refluxing for about 6 hours at 80°C and then filtered using a cotton cloth. The filtrate was then dried using a rota-evaporator at 45 °C under reduced pressure. A sticky powder was obtained and this was termed as aqueous extract. This extract generally comprises about 0.025 to 10% of a mixture of hydroxyanthraquinones and hydroxynaphthoquinones.

### Preferred process for preparation of Phenolic fraction of aqueous extract of Rubia cordifolia:

The aqueous extract (10 g), prepared above was in dissolved in 1 L of 0.1 M NaOH solution. This solution was then extracted with 1 L of ethyl acetate (1 L). The ethyl acetate layer was separated from the mixture. The aqueous layer was neutralized with 1 M HCl and then extracted with chloroform. The chloroform layer was collected and washed with brine solution and then dried using a rotary evaporator. The dry powder thus obtained was termed as the phenolic fraction or hydroxyanthraquinone and hydroxynaphthoquinone rich fraction. About 0.76 g of this fraction was obtained. This extract generally comprises 20 to about 90% of a mixture of hydroxyanthraquinones and hydroxyanpththoquinones.

It is thus possible by way of the extraction process described above to prepare extracts of *Rubia cordifolia* comprising 0.025 to 90% of a mixture of hydroxyanthraquinones and hydroxynapththoquinones. Such extracts are thus preferred to be included in the composition of the invention. The extract of *Rubia cordifolia* is present in 0.1 to 10%by weight of the composition.

*Butea monosperma* (Lam.) is commonly known as Flame of forest and belongs to the family Fabaceae. It is locally known by various names like palas, palash, mutthuga, bijasneha, dhak, khakara, chichra, Bastard Teak, Bengal Kino, and Nourouc in India. It is commonly grown throughout India, Burma and Ceylon except in very arid parts. Generally it grows on open grasslands and scattered in mixed forests. Almost all the parts of the plant are used for a long time in medicinal applications and for other purposes. In the present invention the extract of the flower of the plants is preferred for use in the composition of the invention.

Two types of extracts of *Butea monosperma* are preferred for use in the composition of the invention: Aqueous extract and Enriched extracts.

### Preferred process for preparation of aqueous extract:

100 of dry *Butea monosperma* flowers were extracted with 800 ml water at room temperature under vacuum (800 mbarr) for 24 hours. About 730 ml of aqueous extract was separated from the flowers. The aqueous extract was evaporated to dryness to obtain about 25 g of solids. This extract generally comprises from about 0.1 to 4 weight% butein.

### Preferred process for preparation of Enriched Extracts:

This solid obtained from the aqueous extract prepared above was dissolved in 200 ml water and passed through a column packed with MCI gel. The MCI bed was rinsed with water to remove un-adsorbed sugars. The adsorbed fraction was eluted with ethanol to obtain Enriched fraction-1 with a Butein content of 2%). The Enriched fraction-1 was evaporated to dryness to obtain ∼ 23 g of solids.

This solid was dissolved in 10% ethanolic aqueous solution (500 ml) followed by solvent extraction with diethyl ether to obtain Enriched fraction-2 with a Butein content of 25%). This extract was evaporated to dryness to obtain about 8 g of solid powder. This extract generally comprises about 2 to 30% weight butein.

The composition comprises 0.1 to 10% extract of *Butea monosperma* comprising higher than 0.02 % Butein. Butein has the structure

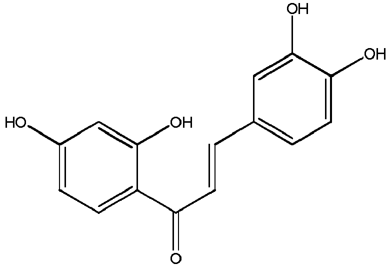

It is thus possible by way of the extraction processes described above to prepare extracts of *Butea monosperma* comprising 0.02 to 30% of butein. Such extracts are thus preferred to be included in the composition of the invention. The extract of *Butea monosperma* comprises higher than 0.02% butein, preferably between 0.1 and 30% by weight of the extract.

Without wishing to be bound by theory, the inventors believe that the combination of the extracts of the two plants as per the invention work through different mechanisms to deliver the desired synergistic benefit. One mechanism is through enhancement of Nitric Oxide production in the desired cells. Nitric oxide (NO) is a vital signaling molecule to influence vascular tone. Enhanced nitric oxide production in endothelial cells via eNOS enzyme are associated with various benefits. These benefits varies with the type of endothelail (micro-endothelial / macro-endothelial) cells. The mechanism of regulation of eNOS enzyme is believed to be the same in both these cell types. Higher NO in micro-endothelial cells results in enhanced microcirculation, enhanced detoxification, enhanced wound healing, reduced post inflammatory hyper-pigmentation, when applied topically. Higher NO in macro-endothelial cells associated with reduced cardiovascular diseases, reduced blood pressure, reduced atherosclerosis and other macro vascular complications. The present inventors have found that when extract of *Rubia cordifolia* with enhanced amount of polyphenols viz. hydroxyanthraquinones and hydroxynaphthoquiniones are used, they activate eNOS thereby increasing the NO production in endothelial cells. This increase in NO production is via eNOS phosporylation. There are several mechanisms which activate this enzyme's phosporylation sites e.g. they may be receptor- ligand interaction or hyperpolarization. The exact detailed mechanism by which this occurs remains unknown. It is important to note that concentrations used in in vitro assays will be significantly lower than the levels used in topical or edible compositions to get similar benefits. The benefits obtained and concentrations of the extracts when used *in vitro* experiments, where the cells are in direct contact with the extracts and actives will be approximately 1000 times lower than in the compositions that are formulated considering the limitations of bio-availability and permeation.

The role of butein is believed to be through a different mechanism. Butein, an ingredient in extract of *Butea monosperma,* does not influence eNOS enzyme. However, it has a role in modulation of phase-II antioxidant enzymes, in reducing Arginase activity, and in increasing free radical scavenging enzyme. This mechanism is known to suppress reactive oxygen species (ROS), which would scavenge NO has it is produced inside the cell. The scavenging mechanism is due to reaction of NO with free radicals such as nacent oxygen or superoxide, which could lead to peroxinitrites formation. Peroxinitrite formation is believed to have a negative implication as it could worsen the situation, by triggering higher superoxides. The present inventors believe that the synergistic benefit obtained by way of the present invention occurs due to the combination of these two mechanisms - one which influences eNOS activity, while the other which influences eradication of other reactive species that may quench NO that is produced.

The composition of the invention may be applied as a topical composition. Such topical composition preferably comprises a cosmetically acceptable base. The cosmetically acceptable base may be selected from a liquid, an emulsion, lotion, cream, foam, gel, soap bar, stick, mask, pad or patch.

The composition of the invention may alternately be formulated as a food composition for oral consumption. The oral composition is preferably formulated as a beverage like soup, tea or coffee or is in non-liquid forms like a spread, a dressing, a dessert, bread; or a solid or powdered food supplement.

According to another aspect of the present invention there is provided a method of improving microcirculation of skin comprising applying to the skin a composition of the invention.

The invention will now be illustrated with the help of the following non-limiting examples.

### EXAMPLES

### Materials used

DAF FM-DA was purchased from Invitrogen (Eugene, Oregon). Dolbeco's Modified Essential Medium (DMEM) were purchased from Sigma (St. Louis, MO, USA), Foetal bovine serum (FBS) was procured from Gibco.

Samples of *Rubia cordifolia* and *Butea monosperma* were sourced from a supplier Channabasappa and Co., from Bangalore, India. The geographical origin of the samples was India, Nepal and Iran in the case of *Rubia cordifolia* and was India in the case of *Butea monosperma.*

### Preparation of culture medium:

### Preparation of Dolbeco's Modified Essestial Medium (DMEM).

This culture medium was prepared by adding 1 vial (13.4 grams) of DMEM (Sigma-Aldrich; Cat no # D5796 SIGMA) in 890 ml of distilled autoclaved water followed by the addition of 100 ml of FBS (Gibco®; Cat no # 16000-044)_and 10 ml of penicillin-streptomycin solution (Sigma-Aldrich; Cat no # P4333 SIGMA).

Human endothelial cell line (EA.hy926, 1X10⁵ cells/10 ml of DMEM) (American Type Culture Collection (ATCC); CRL-2922 - ATCC), was grown in a DMEM growth media (as prepared above) containing 10% v/v FBS (from Gibco) in a CO₂ incubator at 37°C with 5% CO₂ condition.

### Cytotoxicity assay:

Before starting the experiment it was needed to determine the cytotoxic dose for the different extracts. The cytotoxicity of the different extracts was determined as follows by MTT ((3-(4, 5-dimethyl-2-thiazolyl)-2, 5-diphenyl-2H-tetrazolium bromide) assay:
The cultured human endothelial cell line EA.hy926 cells were procured from American Type Culture Collection (CRL-2922 - ATCC) and were cultured in DMEM (Sigma) supplemented with 2mM L-glutamine, 100U/ml penicillin, 100µg/ml streptomycin and 10% vol/vol FBS (Gibco, Invitrogen). Cells were cultured at 37°C in 95% humidified air with 5% CO₂. After attaining 70-80% confluence, the cells were sub-cultured by trypsinization (0.25% Tryp-EDTA, for 2-3 min). For experimental purpose, cells were seeded onto cell culture plates (CLS3524 Sigma).

2x10⁶ of Human endothelial cell line (EaHy926) were suspended in 10 ml of DMEM growth media. 100 µL of this suspension was plated in each well of 96 well flat bottom plate (Thermo Scientific, Nunc; Cat # No.:161093). Therefore each well has a cell concentration of 2x10⁴ cells. This cells were then allowed to adhere for 4hrs in a CO₂ incubator (Thermo Scientific; Forma* Series II 3110 Water-Jacketed CO2 Incubator) at 37°C with 5% CO₂ condition. After 4hrs, different concentration of the extracts in the concentration range of 0.1 -100 µg/ml were prepared in DMEM and was added to the cells and incubated further for 24 hours. To prepare the extracts in the above concentration range, the extracts were first dissolved in DMSO (Dimethyl sulfoxide, Sigma-Aldrich; Cat # D5879) at 50 mg/ml stock concenration. The DMSO stock of the extract were further resuspended in DMEM with the concentration range of 0.1-100 µg/ml. After 24 hours, the cells were washed with phosphate buffered saline (PBS) buffer and treated with 100 µL of MTT reagent (Sigma-Aldrich; Cat # M5655 SIGMA) for 4 hours in a CO₂ incubator. The MTT reagent was prepared by dissolving the powdered MTT in PBS (concentration:5mg/mL). After 4 hrs, the supernatant were removed and MTT formazan crystal formed inside the cells were collected and dissolved in DMSO (Sigma-Aldrich; Cat # D5879) and absorbance was measured at a wavelength of 540nm using Biorad multiplate reader (Bio-Rad; Model 680).

It was observed that the maximum non-cytotoxic concentration was 20 µg/ml for the *Rubia cordifolia* extract and 50 µg/ml for *Butea monosperma* extract when incubated for 24 hours. Therefore for subsequent experimentation, the non-cytotoxic concentration of 20 µg/ml for *Rubia* extract and 50 µg/mL for *Butea* extract or less were used. It was further seen that the mximum non-cytotoxic concentration for *Rubia Cordifolia* extract was 50 µg/ml upto 4 hours.

### Preparation of EA.Hy926 cells for Nitric oxide assay

The cultured human endothelial cell line EA.Hy926 cells were procured from American Type Culture Collection (CRL-2922 - ATCC) and were cultured in DMEM (Sigma) supplemented with 2mM L-glutamine, 100U/ml penicillin, 100µg/ml streptomycin and 10% vol/vol FBS (Gibco, Invitrogen). Cells were cultured at 37°C in 95% humidified air with 5% CO₂. After attaining 70-80% confluence, the cells were sub-cultured by trypsinization (0.25% Tryp-EDTA, for 2-3 min). For experimental purpose, cells were seeded onto 24 well tissue culture plates (Sigma-Aldrich; Z688789 SIGMA). After adherence, cells were subjected to starvation in serum free low glucose DMEM for 12-14 hrs prior to any experiment, to maintain the cells in quiescent state and reduce the basal level of NO production.

### Measurement of intracellular nitri oxide using flow-cytometry.

### Experimental flow:

1. EAHy926 (5x10⁵) cells were seed into 24 well tissue culture plate and allowed to adhere for 12-16 hrs.
2. After adherence, cells were subjected to starvation in serum free low glucose DMEM for 12-14 hrs prior to any experiment.
3. After adherence, cells were treated with or without the extract containing the active A (Butein enriched extract) and incubated for 24 hrs in DMEM without FBS.
4. After 24 hours, the cells in 24 well tissue culture plates were loaded with DAF FM-DA (1µM) for 30 min, washed twice with serum free medium.
5. Subsequently, cells were stimulated with different concentration of active B (Rubia extract) for 45 minutes at 37°C in 95% humidified air with 5% CO₂.
6. The stimulated cells were then washed twice with serum free medium.
7. The cells were trypsinized with 0.25% Trypsin-EDTA and fixed with 2% PFA for 15 min.
8. The suspension of cells were acquired using flow-cytometry. A population of 10,000 cells were gated and their relative fluorescence intensities were measured using FACS Calibur (BD; SanDiego). The fluorescent intensity of cells which reflects the NO production was compared between treated and untreated cells.

The results are tabulated as below in Tbale 1.

**Table 1**

| Examples | Fold change: Mean of three readings | Standard deviation | % butien in the well | % Anthraquinone |
|---|---|---|---|---|
| Untreated Control | 1 | 0 | 0 | 0 |
| Phenolic Rubia extract 20 µg/ml | 4.15 | 1.02 | 0 | 0.0018 |
| Phenolic Rubia extract 10 µg/ml | 2.52 | 1.02 | 0 | 0.0009 |
| Enriched Butea extract 50 µg/ml | 2.44 | 0.91 | 0.0015 | 0 |
| Enriched Butea extract 25 µg/ml | 2.83 | 0.59 | 0.00075 | 0 |
| Mixture of Enriched Butea extract 50 µg/ml and Phenolic Rubia extract 20 µg/ml | 27.17 | 0.18 | 0.0015 | 0.0018 |
| Mixture of Enriched Butea extract 25 µg/ml and Phenolic Rubia extract 20 µg/ml | 17.12 | 1.59 | 0.00075 | 0.0018 |
| Mixture of Enriched Butea extract 50 µg/ml and Phenolic Rubia extract 10 µg/ml | 21.86 | 2.25 | 0.0015 | 0.0009 |
| Mixture of Enriched Butea extract 25 µg/ml and Phenolic Rubia extract 10 µg/ml | 16.85 | 2.39 | 0.00075 | 0.0009 |

In the above table Phenolic *Rubia* extract refers to an extract having about 85% hydroxyanthraquinones and hydroxynaphthoquinones.

Enriched Butea extract refers to an extract having about 25% butein. The actual concentration in each well of the tissue culture plate based on the quantity taken is also indicated in the above table.

The data in the above table indicates that combination of *Butea monosperma* extract and *Rubia cordifolia* extract provides for synergistic activity in an invitro assay indicative of enhanced microcirculation.

### Effect of Concentration of Rubia cordifolia and Butea monosperma extracts:

Effect of various concentrations of *Rubia cordifolia* and *Butea monosperma* extracts were tested using the procedure mentioned above. As the maximum non-cytotoxic concentration for *Rubia cordifolia* extract was 50 µg/ml upto 4 hours the concentration selected for both Rubia and Butea was upto 50 µg/ml in these experiments. The data are presented in Table 2.

**Table 2**

| | *Rubia* (µg/ml) | | | | *Butea* (µg/ml) | | | | NO fold chang e | % butien | % antraquin one |
|---|---|---|---|---|---|---|---|---|---|---|---|
| Control | - | - | - | - | - | - | - | - | 0.95 | 0 | 0 |
| Ex A | 1 | - | - | - | - | - | - | - | 1.02 | 0 | 0.00009 |
| Ex B | - | 10 | - | - | - | - | - | - | 2.86 | 0 | 0.0009 |
| Ex C | - | - | 20 | - | - | - | - | - | 3.50 | 0 | 0.0018 |
| Ex D | - | - | - | 50 | - | - | - | - | 4.68 | 0 | 0.0045 |
| Ex E | - | - | - | - | 1 | - | - | - | 1.01 | 0.00003 | 0 |
| Ex F | - | - | - | - | - | 10 | - | - | 1.23 | 0.0003 | 0 |
| Ex G | - | - | - | - | - | - | 25 | - | 2.67 | 0.00075 | 0 |
| Ex H | - | - | - | - | - | - | - | 50 | 2.75 | 0.0015 | 0 |
| Ex I | 1 | - | - | - | - | 10 | - | - | 1.27 | 0.0003 | 0.00009 |
| Ex J | - | 10 | - | - | - | 10 | - | - | 3.31 | 0.0003 | 0.0009 |
| Ex K | - | - | 20 | - | - | 10 | - | - | 7.25 | 0.0003 | 0.0018 |
| Ex L | - | - | - | 50 | - | 10 | - | - | 13.74 | 0.0003 | 0.0045 |
| Ex M | 1 | - | - | - | - | - | 25 | - | 3.18 | 0.00075 | 0.00009 |
| Ex N | - | 10 | - | - | - | - | 25 | - | 15.65 | 0.00075 | 0.0009 |
| Ex O | - | - | 20 | - | - | - | 25 | - | 19.16 | 0.00075 | 0.0018 |
| Ex P | - | - | - | 50 | - | - | 25 | - | 22.75 | 0.00075 | 0.0045 |
| Ex Q | 1 | - | - | - | - | - | - | 50 | 5.75 | 0.0015 | 0.00009 |
| Ex R | - | 10 | - | - | - | - | - | 50 | 22.62 | 0.0015 | 0.0009 |
| Ex S | - | - | 20 | - | - | - | - | 50 | 27.23 | 0.0015 | 0.0018 |
| Ex T | - | - | - | 50 | - | - | - | 50 | 32.80 | 0.0015 | 0.0045 |
| Ex U | 1 | - | - | - | 1 | - | - | - | 1.25 | 0.00003 | 0.00009 |
| Ex V | - | 10 | - | - | 1 | - | - | - | 2.00 | 0.00003 | 0.0009 |
| Ex W | - | - | 20 | - | 1 | - | - | - | 3.30 | 0.00003 | 0.0018 |
| Ex X | - | - | - | 50 | 1 | - | - | - | 5.35 | 0.00003 | 0.0045 |

The results presented in Tbale 2 show that enhanced or synergistic benefit is obtained when a combination of the extracts of *Butea monosperma* and *Rubia cordifolia* are used as compared to the benefits obtained by using the individual extracts. The benefits are clear when the concentration of anthraquinones are at 0.0009% which corresponds to 10µg/ml of phenolic extract of *Rubia* in combination with 0.0003% of butien which corresponds to 10µg/ml of extract of *Butea.* The benefits obtained and concentrations mentioned in the table are based on *in vitro* experiments, where the cells are in direct contact with the extracts and actives. With limitations of bio-availability and permeation, the concentrations required for observed benefits will be approximately 1000 times in the compositions that are formulated.

At low concentrations of either of the extracts i.e at 1µg/ml of either *Butea* or *Rubia* no synergistic benefits were obtained.

## Claims

1. A topical or oral or parenteral composition for enhanced micro/ macro blood circulation comprising
(i) 0.1 to10 % of an extract *of Rubia Cordifolia* comprising higher than 0.025 % hydroxyanthraquinones and hydroxynaphthoquinones;
(ii) 0.1 to 10 % an extract of *Butea monosperma* comprising higher than 0.02 % butein.

2. A composition as claimed in claim 1 wherein the composition comprises 0.1 to 5% by weight of an extract of *Rubia cordifolia* and 0.3 to 3% by weight of an extract of *Butea monosperma.*

3. A topical composition as claimed in claim 1 additionally comprising a cosmetically acceptable base selected from an emulsion, lotion, cream, foam, gel, soap bar, stick, mask, pad or patch.

4. An oral composition as claimed in claim 1 wherein the composition is a beverage like soup, tea or coffee or is in non-liquid forms like a spread, a dressing, a dessert, a bread; or a solid or powdered food supplement.

5. Composition according to claims 3 or 4 for use in a method of improving blood microcirculation of skin.

## Patentansprüche

1. Topische oder orale oder parenterale Zusammensetzung für verbesserte Mikro/Makro-Blutzirkulation, umfassend
(i) 0,1 bis 10% eines Extrakts von *Rubia Cordifolia,* umfassend mehr als 0,025% Hydroxyanthrachinone und Hydroxynaphthochinone,
(ii) 0,1-10% eines Extrakts von *Butea monosperma,* umfassend mehr als 0,02% Butein.

2. Zusammensetzung, wie in Anspruch 1 beansprucht, wobei die Zusammensetzung 0,1 bis 5 Gewichts-% eines Extrakts von *Rubia cordifolia* und 0,3 bis 3 Gewichts-% eines Extrakts von *Butea monosperma* umfasst.

3. Topische Zusammensetzung, wie im Anspruch 1 beansprucht, die zusätzlich ein kosmetisch annehmbares Grundmaterial umfasst, ausgewählt aus einer Emulsion, Lotion, Creme, einem Schaum, Gel, Seifenstück, Stab, einer Maske, einem Kissen oder Flicken.

4. Orale Zusammensetzung, wie im Anspruch 1 beansprucht, wobei die Zusammensetzung ein Getränk ist, wie eine Suppe, Tee oder Kaffee, oder in nicht-flüssigen Formen, wie einem Aufstrich, Dressing, Dessert, Brot oder festen oder pulverigen Nahrungsmittelergänzungsmittel, vorliegt.

5. Zusammensetzung nach Anspruch 3 oder 4 zur Verwendung in einem Verfahren zur Verbesserung der Blutmikrozirkulation von Haut.

## Revendications

1. Composition topique ou orale ou parentérale pour une micro/macrocirculation sanguine améliorée comprenant :
(i) de 0,1 à 10 % d'un extrait de *Rubia Cordifolia* comprenant plus de 0,025 % d'hydroxyanthraquinones et hydroxynaphtoquinones ;
(ii) de 0,1 à 10 % d'un extrait de *Butea monosperma* comprenant plus de 0,02 % de butéine.

2. Composition selon la revendication 1, dans laquelle la composition comprend de 0,1 à 5 % en masse d'un extrait de *Rubia Cordifolia* et de 0,3 à 3 % en masse d'un extrait de *Butea monosperma.*

3. Composition topique selon la revendication 1 comprenant de plus une base cosmétiquement acceptable choisie parmi une émulsion, une lotion, une crème, une mousse, un gel, une savonnette, un stick, un masque, un tampon ou une pastille.

4. Composition orale selon la revendication 1, dans laquelle la composition est une boisson comme une soupe, du thé ou du café ou est dans des formes non liquides comme une pâte à tartiner, un assaisonnement, un dessert, un pain ; ou un supplémentaire alimentaire solide ou pulvérulent.

5. Composition selon la revendication 3 ou 4 pour une utilisation dans un procédé d'amélioration de la microcirculation sanguine de la peau.
